# EUROPÄISCHE PATENTSCHRIFT

(11) **EP 0 530 696 B1**
(45) Veröffentlichungstag und Bekanntmachung des Hinweises auf die Patenterteilung: **09.08.1995**
(21) Anmeldenummer: 92114711.2
(22) Anmeldetag: 28.08.1992
(51) Int. Cl.: C07C 209/48, C07C 211/09

(54) **Verfahren zur Herstellung von Pentan-1,5-diaminen**
Process for the preparation of pentane 1,5-diamines
Procédé de préparation de pentane-1,5-diamines

(30) Priorität: 04.09.1991 DE 4129350
(43) Veröffentlichungstag der Anmeldung: 10.03.1993
(73) Patentinhaber: BASF Aktiengesellschaft, 67063 Ludwigshafen (DE)
(72) Erfinder: Witzel, Tom, Dr., W-6700 Ludwigshafen (DE); Fuchs, Eberhard, Dr., W-6700 Ludwigshafen (DE); Merger, Franz, Dr., W-6710 Frankenthal (DE); Priester, Claus-Ulrich, Dr., W-6700 Ludwigshafen (DE)

(56) Entgegenhaltungen:
- EP-A- 0 010 179
- EP-A- 0 042 119
- EP-A- 0 077 105
- FR-A- 2 292 698
- CHEMISCHE BERICHTE Bd. 99, 1966, WEINHEIM DE Seiten 3387 - 3389 G. VITA, G. BUCHER 'Notiz zur Darstellung aliphatischer Diamine'

## Beschreibung

Die vorliegende Erfindung betrifft ein neues Verfahren zur Herstellung von aliphatischen Pentan-1,5-diaminen aus gamma-Cyanoketonen sowie neue Pentan-1,5-diamine.

Aus Chem. Ber. 3387 bis 3389 (1966) ist die Herstellung von 1,5-Diaminohexan aus 5-Oxocapronitril bekannt. Hiernach erhält man 1,5-Diaminohexan nur über die Zwischenstufe des Oxims, indem man zunächst 5-Oxocapronitril mit Hydroxylaminhydrochlorid in Gegenwart von wasserfreiem Natriumcarbonat zum 5-Oximinocapronitril umsetzt und dieses anschließend mit Lithiumaluminiumhydrid zum Diamin reduziert; die Gesamtausbeute beträgt 42 %. Direkte aminierende Hydrierung des 5-Oxo-capronitrils führt nach Angaben der Autoren zum Cyclisierungsprodukt, dem 2-Methylpiperidin.

Die grundsätzliche Problematik der Cyclisierung zu Piperidinen bei der aminierenden Hydrierung von γ-Cyanoketonen ist in Houben-Weyl, Band 11/1, Seite 357 bis 359, beschrieben.

Aus der EP-A-42 119 ist ein Verfahren zur Herstellung primärer Mono- und Diamine aus Oxoverbindungen, die gegebenenfalls auch weitere zur Reduktion befähigte Gruppen enthalten können, mit Ammoniak und Wasserstoff in Gegenwart bekannter Hydrierkatalysatoren beschrieben, bei dem man vor der Reaktion mit Ammoniak und Wasserstoff in Gegenwart von Hydrierkatalysatoren die Oxo-Verbindungen bei Temperaturen von 10 bis 200°C und Drücken von 1 bis 300 bar einer Vorreaktion mit Ammoniak in Gegenwart von anorganischen und organischen Ionenaustauschern in der Ammoniumform als Iminbildungskatalysatoren unterwirft. Die Anwendung des Verfahrens ist ausschließlich in den Beispielen für die aminierende Hydrierung von 3-Cyano-3,5,5-trimethyl-cyclohexanon (Isophoronnitril) und 2,2,6,6-Tetramethyl-4-piperidon (Triacetonamin) beschrieben. Bei der aminierenden Hydrierung von Isophoronnitril werden durch den Einsatz des organischen Ionenaustauschers Lewatit SPR 120 in der Iminierung geringfügige Ausbeuteverbesserungen gegenüber der nicht-katalysierten Fahrweise erzielt (vgl. Vergleichsbeispiel 3 in der EP-A-42 119: 90,3 % Ausbeute, mit Lewatit SPR 120: 93,9 bis 94,7 %).

Es steht also bislang kein Verfahren zur Verfügung, nach dem offenkettige γ-Cyanoketone unter technisch praktikablen Bedingungen und mit wirtschaftlich befriedigenden Ausbeuten in die entsprechenden offenkettigen Pentan-1,5-diamine überführt werden können.

Der vorliegenden Erfindung lag daher die Aufgabe zugrunde, ein Verfahren zur Herstellung von Pentan-1,5-diaminen zu finden, das den zuvor genannten Nachteilen abhilft, insbesondere aus γ-Cyanoketonen unter technisch praktikablen Bedingungen Pentan-1,5-diamine herzustellen, mit technisch befriedigende Ausbeuten bzw. Raum-Zeit- Ausbeuten und neue Pentan-1,5-diamine mit - im Vergleich zum bekannten 1,5-Hexandiamin - höherem Substitutionsgrad herzustellen.

Demgemäß wurde ein neues und verbessertes Verfahren zur Herstellung von Pentan-1,5-diaminen der allgemeinen Formel I 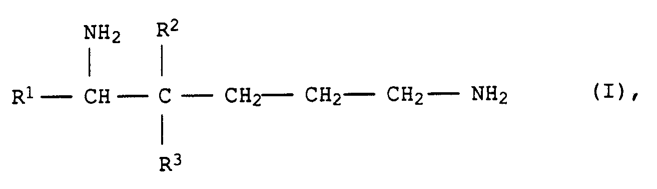
in der
- R¹: C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl und
- R², R³: unabhängig voneinander Wasserstoff, C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substituiert ist, C₂- bis C₂₀-Alkenyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl bedeuten oder gemeinsam für eine gegebenfalls durch eine bis fünf C₁-bis C₄-Alkylgruppen substituierte C₄- bis C₇-Alkylenkette stehen,
aus γ-Cyanoketonen der allgemeinen Formel II 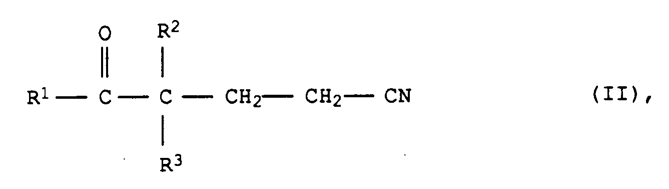
in der die Substituenten R¹, R² und R³ die oben genannten Bedeutungen haben, welches dadurch gekennzeichnet ist, daß man in zwei räumlich voneinander getrennten Reaktionsräumen
a) die Cyanoketone der Formel II in einem ersten Reaktionsraum mit überschüssigem Ammoniak an aciden Heterogenkatalysatoren bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an Cobalt-, Nickel-, Ruthenium-, Palladium- und/oder anderen edelmetallhaltigen Katalysatoren, gegebenenfalls mit basischen Komponenten oder auf basischen oder neutralen Trägern bei Temperaturen von 50 bis 180°C und Drücken von 30 bis 500 bar hydriert.

Ferner wurden neue Pentan-1,5-diamine der allgemeinen Formel I' 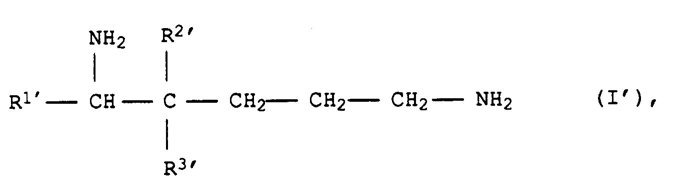
in der
- R¹': C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₂- bis C₂₀-Alkinyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl und
- R²', R³': unabhängig voneinander Wasserstoff, C₁- bis C₂₀-Alkyl, das gegebenenfalls durch ein bis vier heterocyclische Reste, C₂- bis C₈-Carbalkoxy, Carboxy, C₁- bis CAlkylamino und/oder Hydroxy substituiert ist, C₂- bis C₂₀-Alkenyl, C₂- bis C₂₀-Alkinyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl bedeuten oder gemeinsam für eine gegebenfalls durch eine bis fünf C₁- bis C₄-Alkylgruppen substituierte C₄- bis C₇-Alkylenkette stehen,
mit der Maßgabe, daß R¹' nicht Methyl bedeutet, wenn R²' und R³' gleichzeitig Wasserstoff bedeuten.

Das erfindungsgemäße Verfahren läßt sich wie folgt in zwei räumlich voneinander getrennten Reaktionsräume durchführen:
a) In einer ersten Verfahrensstufe setzt man die gamma-Cyanoketone mit überschüssigem Ammoniak um. Dabei hält man einen Druck von 15 bis 500 bar, vorzugsweise von 30 bis 350 bar und eine Temperatur von 20 bis 150°C, vorzugsweise von 30 bis 100°C ein. Die Kondensation wird in Gegenwart von aciden Heterogenkatalysatoren durchgeführt.
   Als acide Heterogenkatalysatoren eignen sich Metallverbindungen mit Lewissäuren- oder Bröstedtsäure-Charakter wie z. B. Aluminiumoxid, Siliciumdioxid, Titandioxid, Zirkondioxid, ferner Phosphate, wie z. B. Aluminiumphosphate oder Silikate wie z. B. amorphe oder kristalline Alumosilikate. Bevorzugt verwendet man Aluminiumoxid, Titandioxid, Zirkondioxid und Siliciumdioxid, insbesondere Aluminiumoxid und Titandioxid. Die Acidität der Katalysatoren kann gegebenenfalls durch Dotierung mit Halogeniden erhöht werden. So finden z. B. auch halogendotierte Katalysatoren, wie Chlorid auf Aluminiumoxid oder Chlorid auf Titandioxid, Verwendung.
   Bei der Umsetzung der γ-Cyanoketone an den aciden Heterogenkatalysatoren hält man eine Katalysatorbelastung von 0,01 bis 10, vorzugsweise 0,05 bis 7, besonders bevorzugt von 0,1 bis 5 kg Cyanoketon pro kg Katalysator und Stunde ein. Pro Mol Cyanoketon setzt man zweckmäßig, aber nicht zwingend 5 bis 500 Mol NH₃, bevorzugt 10 bis 400, besonders bevorzugt 20 bis 300 Mol ein. Die Umsetzung der gamma-Cyanoketone mit Ammoniak kann auch in der Anwesenheit von inerten Lösungsmitteln, wie Alkanolen oder Tetrahydrofuran erfolgen.
   Die Umsetzung der γ-Cyanoketone läßt sich diskontinuierlich, bevorzugt kontinuierlich durchführen, z. B. in Druckbehältern oder Druckbehälterkaskaden. Nach einer besonders bevorzugten Ausführungsform werden die γ-Cyanoketone und NH₃ durch einen Rohrreaktor geleitet, in dem der Katalysator in Form eines festen Bettes angeordnet ist.
b) Das so erhaltene Produkt wird in einer zweiten Verfahrensstufe mit 3 bis 10 000 Moläquivalenten Wasserstoff, bevorzugt 4 bis 500, besonders bevorzugt 4.5 bis 200, gegebenenfalls nach Zufuhr von weiterem Ammoniak, einer katalytischen Hydrierung zugeführt.
   Die Hydrierung wird vorzugsweise in flüssigem Ammoniak durchgeführt. Pro Mol in Stufe 1 eingesetztem gamma-Cyanoketon verwendet man 5 bis 500 Mol NH₃, bevorzugt 10 bis 400 Mol, besonders bevorzugt 20 bis 300 Mol. Der NH₃-Anteil kann gegebenenfalls durch Zufuhr von NH₃ auf den gewünschten Wert erhöht werden.
   Man hydriert im allgemeinen bei einer Temperatur von 50 bis 180°C, bevorzugt von 60 bis 160°C, besonders bevorzugt von 70 bis 140°C und einem Druck von 30 bis 500 bar, bevorzugt von 50 bis 350 bar, besonders bevorzugt von 70 bis 300 bar.
   Die Katalysatorbelastungen liegen zweckmäßig im Bereich von 0,01 bis 5 kg/(kg x h), bevorzugt bei 0,02 bis 2,5, besonders bevorzugt bei 0,05 bis 2 kg/(kg x h).
   Bei der Hydrierung können prinzipiell alle gängigen Hydrierkatalysatoren eingesetzt werden, die Nickel, Cobalt, Eisen, Kupfer, Ruthenium, Palladium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z. B. Aluminiumoxid, Titandioxid, Zirkondioxid, Zinkoxid oder Magnesiumoxid/Aluminiumoxide in Frage, bevorzugt werden Hydrierkatalysatoren mit basischen Komponenten, wie Oxide und Hydroxide von Alkali- und Erdalkalimetallen. Besonders bevorzugt als basische Komponente sind Oxide bzw. Hydroxide der Alkalimetalle, wie z.B. die des Natriums. Die basische Komponente kann ggf. auch während des Hydrierprozesses zugeführt werden, z. B. als Lösung von Alkali- oder Erdalkalihydroxiden in Wasser.
   Besonders bevorzugt verwendet man in der Hydrierung Cobalt, Nickel oder Ruthenium mit basischer Komponente.
   Die Umsetzung führt man bevorzugt kontinuierlich, z. B. in druckfesten Rührbehältern oder in einer Rührbehälterkaskade durch.
   In einer besonders bevorzugten Ausführungsform werden Rohrreaktoren eingesetzt, in denen das Hydriergut in Sumpf- oder Rieselfahrweise über ein fest angeordnetes Katalysatorbett geleitet wird.

Die Verfahrensstufen a und b können ebenfalls in einem Reaktor durchgeführt werden, in dem Iminierungskatalysatoren und Hydrierkatalysatoren in zwei getrennten Schichten angeordnet sind. In diesem Fall führt man die Iminierung zweckmäßigerweise in Gegenwart von Wasserstoff durch.

Nach der Hydrierung wird überschüssiges Ammoniak gegebenenfalls unter Druck abgetrennt. Die so erhaltenen Pentan-1,5-diamine lassen sich durch fraktionierende Destillation isolieren. Substituierte Piperidine entstehen nur in untergeordnetem Maße als Nebenprodukt.

Die Ausgangsstoffe für das Verfahren, die γ-Cyanoketone, sind z.B. aus Ketonen und Acrylnitril zugänglich.

Das erfindungsgemäße Verfahren ermöglicht hiermit die Umwandlung der γ-Cyanoketone in hohen Ausbeuten und Raum-Zeit-Ausbeuten zu den Pentan-1,5-diaminen.

Die Substituenten R¹, R² und R³ in den Verbindungen I und II haben folgende Bedeutungen:
- R¹, R², R³: - unverzweigtes oder verzweigtes C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- unverzweigtes oder verzweigtes C₁- bis C₂₀-Alkyl, das durch ein bis vier, bevorzugt ein bis drei, besonders ein oder zwei heterocyclische Reste, wie Pyridyl, und/oder C₂- bis C₈-Carbalkoxy, Carboxy, C₁- bis C₈-Alkylamino und/oder Hydroxy substituiert ist, bevorzugt Heteroaryl, besonders bevorzugt Pyridyl,
- C₂- bis C₂₀-Alkenyl, bevorzugt C₂- bis C₈-Alkenyl, besonders bevorzugt C₂- bis C₄-Alkenyl wie Vinyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl;
- C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopropyl, Cyclopentyl, Cyclohexyl und Cyclooctyl,
- C₄- bis C₂₀-Cycloalkyl-alkyl, bevorzugt C₄- bis C₁₂-Cycloalkyl-alkyl, besonders bevorzugt C₄- bis C₈-Cyclo-alkyl-alkyl wie Cyclopentylmethyl und Cyclohexylmethyl;
- C₄- bis C₂₀-Alkyl-cycloalkyl, bevorzugt C₄- bis C₁₂-Alkyl-cycloalkyl, besonders bevorzugt C₄- bis C₈-Alkyl-Cycloalkyl wie Methyl-cyclopentyl und Methylcyclohexyl;
- C₁- bis C₂₀-Alkoxyalkyl, bevorzugt C₁- bis C₈-Alkoxyalkyl, besonders bevorzugt C₁- bis C₅-Alkoxyalkyl wie Methoxymethyl, Ethoxymethyl, n-Propoxymethyl, iso-Propoxy-methyl, n-Butoxymethyl, iso-Butoxymethyl, sec.-Butoxymethyl, tert.-Butoxymethyl und 1-Methoxy-ethyl,
- C₁- bis C₂₀-Halogenalkyl, bevorzugt C₁- bis C₈-Halogenalkyl, besonders bevorzugt C₁- bis C₄-Fluor- und/oder Chloralkyl wie Fluormethyl, Difluormethyl, Trifluormethyl, Chlormethyl, Dichlormethyl und Trichlormethyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethyl-phenyl, 2,5-Dimethylphenyl, 2,6-Dimethylphenyl, 3,4-Dimethyl-phenyl, 3,5-Dimethylphenyl, 2,3,4-Trimethylphenyl, 2,3,5-Tri-methylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₂- bis C₈-Alkoxycarbonyl, bevorzugt C₁- bis C₄-Alkoxycarbonyl, besonders bevorzugt Methoxy und Ethoxycarbonyl.
- R² und R³: zusätzlich
unabhängig voneinander
- Wasserstoff
- gemeinsam für eine gegebenenfalls durch bis zu fünf C₁- bis C₄-Alkylgruppen substituierte cyclische oder acyclische C₁- bis C₇-Alkylenkette, wie z.B. =CH₂, =CH-CH₃, =C(CH₃)₂, -CH₂-CH₂-CH₂-, -(CH₂)₄-, -(CH₂)₅-, -(CH₂)₆-.

Die Substituenten R¹' bis R³' in den Verbindungen I' haben die gleiche Bedeutung wie die Substituenten R¹ bis R³ in den Verbindungen I, mit der Maßgabe, daß R¹' nicht für Methyl steht, wenn R²' und R³' für Wasserstoff ist.

Bevorzugte γ-Cyanoketone der allgemeinen Formel II sind z.B.:
5-Oxohexannitril (aus Aceton), 4-Methyl-5-oxo-hexannitril (aus Methylethylketon), 4-Methyl-5-oxo-heptannitril (aus Diethylketon), 4-Ethyl-5-oxo-hexannitril (aus Methylpropylketon), 4,4-Dimethyl-5-oxo-hexannitril (aus Methylisopropylketon), 4,4-Dimethyl-5-oxo-heptannitril (aus Ethylisopropylketon), 4,4,5-Trimethyl-5-oxo-heptannitril (aus Diisopropylketon), 4-Isopropyl-5-oxo-hexannitril (aus Methylisobutylketon), 4-Isopropenyl-5-oxo-hexannitril, 4-Isopropyliden-5-oxo-hexannitril (aus Methylisobutenylketon), 6,6-Dimethyl-5-oxo-heptannitril (aus Pinakolon), 4-Decyl-5-oxohexannitril (aus Methylundecylketon), 4-(3-Methyl-butyl)-5-oxo-hexannitril (aus 6-Methyl-2-heptanon), 4-iso-Butyl-5-oxo-hexannitril (aus 5-Methyl-2-hexanon), 4-Butyl-5-oxo-hexannitril (aus 2-Heptanon), 4-(2-Methyl-butyl)-5-oxo-hexannitril (aus 5-Methyl-2-heptanon), 5-Phenyl-5-oxo-pentannitril (aus Acetophenon), 4-Benzyl-5-oxo- hexannitril (aus 4-Phenyl-2-butanon), 4,6-Diphenyl-5-oxo-hexannitril (aus Dibenzylketon), 1-Acetyl-1-cyanethyl-cyclohexan (aus Cyclohexylmethylketon), 1-Acetyl-1-cyanethyl-cyclopropan (aus Cyclopropylmethylketon), 4,4-Dimethoxy-5-oxo-hexannitril (aus Methylglyoxaldimethylacetal), 4-Methoxy-5-oxo-hexannitril (aus Methoxyaceton), 5-Pyridyl-5-oxo-pentannitril (aus Acetylpyridin), 5-Methoxycarbonyl-5-oxo-pentannitril (aus Brenztraubensäuremethylester), 4-Methoxycarbonyl-5-oxo-hexannitril (aus Acetessigester).

Bevorzugte Pentan-1,5-diamine der Formeln I und I' sind: Hexan-1,5-diamin, 4-Methyl-hexan-1,5-diamin, 4-Methyl-heptan-1,5-diamin, 4-Ethyl-hexan-1,5-diamin, 4,4-Dimethyl-hexan-1,5-diamin, 4,4-Dimethyl-heptan-1,5-diamin, 4,4,6,Trimethyl-heptan-1,5-diamin, 4-Isopropyl-hexan-1,5-diamin, 6,6-Dimethyl-heptan-1,5-diamin, 4-Decyl-hexan-1,5-diamin, 4-(3-Methyl-butyl)-hexan-1,5-diamin, 4-iso-Butyl-hexan-1,5-diamin, 4-Butyl-hexan-1,5-diamin, 4-(2-Methyl-butyl)-hexan-1,5-diamin, 1-Phenyl-pentan-1,5-diamin, 4-Benzyl-hexan-1,5-diamin, 4,6-Diphenyl-hexan-1,5-diamin, 1-(1-Aminoethyl)-1-(3-aminopropyl)-cyclohexan, 1-(1-Aminoethyl)-1-(3-aminopropyl)-cyclopropan, 4,4-Dimethoxy-hexan-1,5-diamin, 4-Methoxy-hexan-1,5-diamin, 5-Pyridyl-pentan-1,5-diamin, 5-Methoxycarbonyl-pentan-1,5-diamin, 4-Methoxycarbonyl-hexan- 1,5-diamin.

Die beanspruchten Diamine zeichnen sich gegenüber dem bekannten 1,5-Hexandiamin auf Grund des höheren Substitutionsgrades durch geringere Flüchtigkeit und stärkere Asymmetrie (unterschiedliche Reaktivität der Aminfunktionen) aus. Daraus resultieren u.a. leichte Verarbeitbarkeit der Diamine, beispielsweise als Komponenten (Härter) für Epoxide und Komponenten für Polyamide und geringere Geruchsbelästigungen durch unumgesetzte Diamine sowie der daraus herstellbaren Diisocyanate.

### Beispiele

### Beispiel 1

Ein vertikaler Rohrreaktor (Durchmesser: 16 mm, Füllhöhe: 50 cm, ölbeheizter Doppelmantel) wurde mit 90,1 g (87 ml) eines Katalysators mit 3 % Ruthenium auf β-Aluminiumoxid in Form von 1,2 mm-Strängen gefüllt (Katalysatorherstellung durch Porentränkung von β-Aluminiumoxid mit wäßriger Rutheniumnitrat-Lösung und Trocknen bei 120°C). Der Katalysator wurde zur Reduktion bei 100 bar unter gleichzeitigem Durchleiten von 150 Nl/h Wasserstoff nach schrittweiser Erhöhung der Temperatur von 100 auf 220°C innerhalb von 7 h 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 65.3 g (100 ml) Titandioxid (Anatas) in Form von 1.5 mm-Strängen gefüllt war, wurden bei einem Druck von 250 bar und einer Temperatur von 80°C stündlich 20.5 g 5-Oxo-hexannitril (Reinheit 97.7 %) und 240 g flüssiger Ammoniak gepumpt. Der Austrag wurde anschließend bei einem Druck von 250 bar und einer Temperatur von 110°C unter gleichzeitigem Durchleiten von 100 Nl/h Wasserstoff von unten nach oben durch den Hydrierreaktor geleitet. Nach Entspannen auf Normaldruck wurde NH₃ abdestilliert und der Hydrieraustrag mit quantitativer Gaschromatographie analysiert; es ergab sich eine Ausbeute an 1,5-Hexandiamin von 87 %, die Ausbeute an 2-Methylpiperidin betrug 8 %.

### Beispiel 2

Ein vertikaler Rohrreaktor (Durchmesser: 16 mm, Füllhöhe: 50 cm, ölbeheizter Doppelmantel) wurde mit 177 g (100 ml) eines basischen Cobalt-Vollkontaktes (CoO mit 5 % Mn₂O₃, 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser:. 16 mm, Füllhöhe: 50 cm, ölbeheizter Doppelmantel), der mit 70.0 g (100 ml) γ-Aluminiumoxid in Form von 1.5-mm-Strängen gefüllt war, wurden bei einem Druck von 250 bar und einer Temperatur von 80°C stündlich von unten nach oben 20,5 g 5-Oxo-hexannitril (Reinheit: 97,7 %) und 100 g flüssiger Ammoniak gepumpt. Anschließend wurden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wurde der Ammoniak abdestilliert. Laut gaschromatographischer Analyse des Hydrieraustrages betrug die Ausbeute an 1,5-Hexandiamin 96 %, 2-Methylpiperidin entstand mit 2 % Ausbeute.

### Beispiel 3

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes (CoO mit 5 % Mn₂0₃, 1,4 % Na₂O) in Form von 1 bis 1,5 mm Split gefüllt und wie in Beispiel 2 reduziert.

Durch einen vor den Hydrierreaktor geschalteten Rohrreaktor (Durchmesser: 16 mm, Füllhöhe: 20 cm, ölbeheizter Doppelmantel), der mit 25.4 g (40 ml) Titandioxid (Anatas) in Form von 1,5-mm-Strängen gefüllt war, wurden bei einem Druck von 250 bar und einer Temperatur von 80°C stündlich von unten nach oben 20,5 g 5-Oxo-hexannitril (Reinheit: 97,7 %) und 340 g flüssiger Ammoniak gepumpt. Anschließend wurden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wurde der Ammoniak abdestilliert und der Hydrieraustrag aus 75 Stunden durch fraktionierende Destillation über eine 30 cm Füllkörperkolonne (3 mm Glasringe) aufgetrennt. Mann erhält 1400 g 1,5-Hexandiamin, entsprechend einer Ausbeute von 89 %.

### Beispiel 4

Ein vertikaler Rohrreaktor (Durchmesser: 16 mm, Füllhöhe: 50 cm, ölbeheizter Doppelmantel) wurde mit 177 g (100 ml) eines basischen Cobalt-Vollkontaktes (CoO mit 5 % Mn₂O₃, 1,4 % Na₂O) in Form von 1 bis 1,5 mm Split gefüllt und wie in Beispiel 2 reduziert.

Durch einen vor den Hydrierreaktor geschalteten Rohrreaktor (Durchmesser: 0. 16 mm, Füllhöhe: 50 cm, ölbeheizter Doppelmantel), der mit 63.5 g (100 ml) Titandioxid (Anatas) in Form von 1.5-mm-Strängen gefüllt war, wurden bei einem Druck von 250 bar und einer Temperatur von 80°C stündlich von unten nach oben 21 g 4,4-Dimethyl-5-oxo-hexannitril (Reinheit: 97,2 %) und 180 g flüssiger Ammoniak gepumpt. Anschließend wurden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wurde der Ammoniak abdestilliert. Der Austrag aus 24 Stunden wurde durch fraktionierende Destillation über eine 30 cm Füllkörperkolonne (3 mm Glasringe) aufgetrennt. Man erhält 445 g 4,4-Dimethyl-1,5-hexandiamin als farblose Flüssigkeit (Sdp. 90°C/3 mbar), entsprechend einer Ausbeute von 88 %.

### Beispiel 5

Ein vertikaler Rohrreaktor (Durchmesser: 16 mm, Füllhöhe: 50 cm, ölbeheizter Doppelmantel) wurde mit 177 g (100 ml) eines basischen Cobalt-Vollkontaktes (CoO mit 5 % Mn₂O₃, 1,4 % Na₂O) in Form von 1 bis 1,5 mm Split gefüllt und wie in Beispiel 2 reduziert.

Durch einen vor den Hydrierreaktor geschalteten Rohrreaktor (Durchmesser: 16 mm, Füllhöhe: 50 cm, ölbeheizter Doppelmantel), der mit 63.5 g (100 ml) Titandioxid (Anatas) in Form von 1.5-mm-Strängen gefüllt war, wurden bei einem Druck von 250 bar und einer Temperatur von 80°C stündlich von unten nach oben 10 g 4,4-Dimethyl-5-oxo-hexannitril (Reinheit: 97,2 %) und 195 g flüssiger Ammoniak gepumpt. Anschließend wurden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wurde der Ammoniak abdestilliert und der Hydrieraustrag mittels gaschromatographie quantitativ analysiert; es ergab sich eine Ausbeute von 93.0 % an 4,4-Dimethyl-1,5-hexandiamin und 4.5 % an 2,3,3-Trimethylpiperidin.

### Beispiel 6

Ein vertikaler Rohrreaktor (Durchmesser: 16 mm, Füllhöhe: 50 cm, ölbeheizter Doppelmantel) wurde mit 177 g (100 ml) eines basischen Cobalt-Vollkontaktes (CoO mit 5 % Mn₂O₃, 1,4 % Na₂O) in Form von 1 bis 1,5 mm Split gefüllt und wie in Beispiel 2 reduziert. Durch einen vor den Hydrierreaktor geschalteten Rohrreaktor (Durchmesser: 16 mm, Füllhöhe: 50 cm, ölbeheizter Doppelmantel), der mit 63.5 g (100 ml) Titandioxid (Anatas) in Form von 1.5-mm-Strängen gefüllt war, wurden bei einem Druck von 250 bar und einer Temperatur von 80°C stündlich von unten nach oben 10 g 4-Isopropyl-5-oxo-hexannitril (Reinheit: 95,7 %) und 115 g flüssiger Ammoniak gepumpt. Anschließend wurden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wurde der Ammoniak abdestilliert und der Austrag aus 72 Stunden durch fraktionierende Destillation über eine 30 cm Füllkörperkolonne (3 mm Glasringe) aufgetrennt. Man erhält 640 g 4-Isopropyl-1,5-hexandiamin als farblose Flüssigkeit (Sdp. 80°C/ 1 mbar), entsprechend einer Ausbeute von 90 %.

## Patentansprüche

1. Verfahren zur Herstellung von Pentan-1,5-diaminen der allgemeinen Formel I 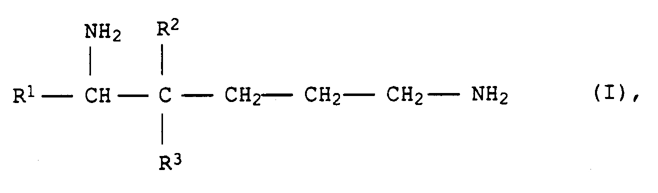 in der
R¹ C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl und
R², R³ unabhängig voneinander Wasserstoff, C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl bedeuten oder gemeinsam für eine gegebenfalls durch eine bis fünf C₁-bis C₄-Alkylgruppen substituierte C₄- bis C₇-Alkylenkette stehen,
aus γ-Cyanoketonen der allgemeinen Formel II 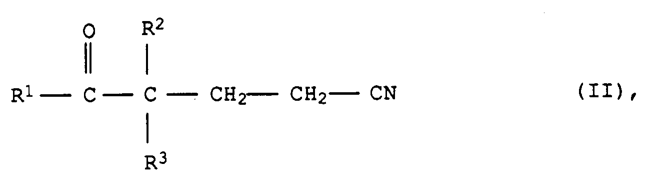 in der die Substituenten R¹, R² und R³ die oben genannten Bedeutungen haben, dadurch gekennzeichnet, daß man in zwei räumlich voneinander getrennten Reaktionsräumen
a) die Cyanoketone der Formel II in einem ersten Reaktionsraum mit überschüssigem Ammoniak an aciden Heterogenkatalysatoren bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an Cobalt-, Nickel-, Ruthenium-, Palladium- und/oder anderen edelmetallhaltigen Katalysatoren, gegebenenfalls mit basischen Komponenten oder auf basischen oder neutralen Trägern bei Temperaturen von 50 bis 180°C und Drücken von 30 bis 500 bar hydriert.

2. Pentan-1,5-diamine der allgemeinen Formel I' 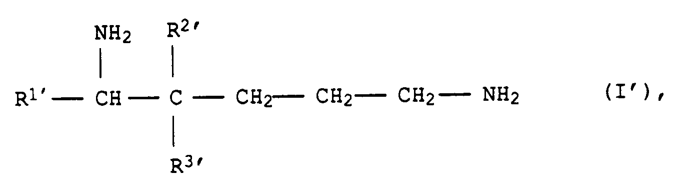 in der
R¹' C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₃- bis C₂₀-Cycloalkyl, C₄- bis C₂₀-Cycloalkyl-alkyl, C₄- bis C₂₀-Alkyl-cycloalkyl, C₂- bis C₂₀-Alkoxyalkyl, C₂- bis C₈-Alkoxycarbonyl, C₁- bis C₂₀-Halogen-alkyl, Aryl, C₇- bis C₂₀-Aralkyl, C₇- bis C₂₀-Alkylaryl und
R²', R³' unabhängig voneinander Wasserstoff, C₁- bis C₂₀-Alkyl, das gegebenenfalls durch ein bis vier heterocyclische Reste, C₂- bis C₈-Carbalkoxy, Carboxy, C₁- bis C₈-Alkylamino und/oder Hydroxy substiuiert ist, C₂- bis C₂₀-Alkenyl, C₃- bis C₂₀- Cycloalkyl, C₄- bis C₂₀-Cycloalkyl-alkyl, C₄- bis C₂₀-Alkylcycloalkyl, C₂- bis C₂₀-Alkoxyalkyl, C₂- bis C₈-Alkoxycarbonyl, C₁- bis C₂₀-Halogenalkyl, Aryl, C₇- bis C₂₀-Aralkyl, C₇- bis C₂₀-Alkylaryl bedeuten oder gemeinsam für eine gegebenfalls durch eine bis fünf C₁- bis C₄-Alkylgruppen substituierte C₄- bis C₇-Alkylenkette stehen,
mit der Maßgabe, daß R¹' nicht Methyl bedeutet, wenn R²' und R³' gleichzeitig Wasserstoff bedeuten.

3. Pentan-1,5-diamine der allgemeinen Formel I' nach Anspruch 2, in der R¹' C₁- bis C₄-Alkyl und R²' und R³' unabhängig voneinander Wasserstoff oder C₁- bis C₈-Alkyl bedeuten, mit der Maßgabe, daß R²' und R³' nicht gleichzeitig Wasserstoff sind, wenn R¹' Methyl bedeutet.

4. Pentan-1,5-diamine der allgemeinen Formel I' nach Anspruch 2, in der R¹' Ethyl, Propyl, iso-Propyl, n-Butyl, sec.-Butyl, iso-Butyl oder tert.-Butyl und R²' und R³' unabhängig voneinander Wasserstoff oder C₁- bis C₈-Alkyl bedeuten.

5. Pentan-1,5-diamine der allgemeinen Formel I' nach Anspruch 2, in der R¹' Methyl und R²' und R³' unabhängig voneinander Wasserstoff oder C₁- bis C₈-Alkyl bedeuten, mit der Maßgabe, daß R²' und R³' nicht gleichzeitig Wasserstoff bedeuten.

6. Verwendung der Pentan-1,5-diamine I nach Anspruch 1 als Härter für Epoxiharze.

7. Verwendung der Pentan-1,5-diamine I' nach den Ansprüchen 2 bis 4 als Härter für Epoxiharze.

8. Verwendung der Pentan-1,5-diamine I nach Anspruch 1 als Komponenten für Polyamide.

9. Verwendung der Pentan-1,5-diamine I' nach Ansprüchen 2 bis 4 als Komponenten für Polyamide.

## Claims

1. A process for the preparation of pentane-1,5-diamines of the formula I 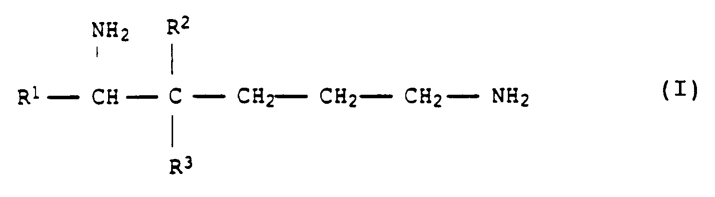 where
R¹ is C₁- to C₂₀-alkyl, which is unsubstituted or substituted by one to four heterocyclic radicals or by C₂- to C₈-carbaloxy, carboxyl, C₁- to C₈-alkylamino and/or hydroxyl, or is C₂-to C₂₀-alkenyl, C₃- to C₂₀-cycloalkyl, C₄- to C₂₀-cycloalkylalkyl, C₄- to C₂₀-alkylcycloalkyl, C₂-to C₂₀-alkoxyalkyl, C₂- to C₈-alkoxycarbonyl, C₁-to C₂₀-haloalkyl, aryl, C₇- to C₂₀-aralkyl or C₇-to C₂₀-alkylaryl, and
R² and R³, independently of one another, are hydrogen, C₁-to C₂₀-alkyl, which is unsubstituted or substituted by one to four heterocyclic radicals or by C₂- to C₈-carbalkoxy, carboxyl, C₁- to C₈-alkylamino and/or hydroxyl, or are C₂- to C₂₀-alkenyl, C₃- to C₂₀-cycloalkyl, C₄- to C₂₀-cycloalkylalkyl, C₄- to C₂₀-alkylcycloalkyl, C₂-to C₂₀-alkoxyalkyl, C₂- to C₈-alkoxycarbonyl, C₁- to C₂₀-haloalkyl, aryl, C₇- to C₂₀-aralkyl or C₇- to C₂₀-alkylaryl, or together are a C₄- to C₇-alkylene chain which is unsubstituted or substituted by one to five C₁- to C₄-alkyl groups,
from γ-cyanoketones of the formula II 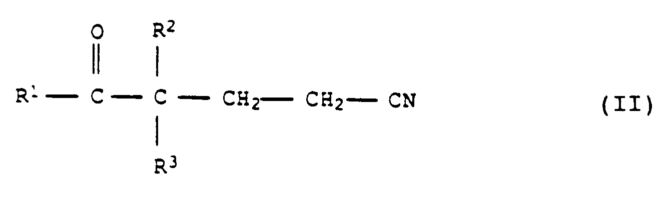 where R¹, R² and R³ are as defined above,
which comprises
a) reacting the cyanoketones of the formula II with excess ammonia in a first reaction space on acidic heterogeneous catalysts at from 20 to 150°C and at from 15 to 500 bar, and
b) hydrogenating the resultant reaction products using hydrogen in a second reaction space in the presence of excess ammonia on cobalt-, nickel-, ruthenium-, palladium- and/or other noble metal-containing catalysts, with or without basic components, unsupported or on basic or neutral supports, at from 50 to 180°C and at from 30 to 500 bar,
in two spatially separate reaction spaces.

2. A pentane 1,5-diamine of the formula I' 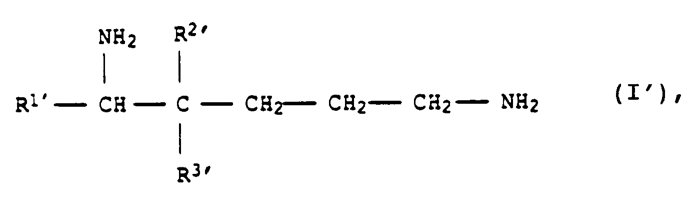 where
R^{1'} is C₁- to C₂₀-alkyl, which is unsubstituted or substituted by one to four heterocyclic radicals or by C₂- to C₈-carbalkoxy, carboxyl, C₁- to C₈-alkylamino and/or hydroxyl, or is C₂-to C₂₀-alkenyl, C₃- to C₂₀-cycloalkyl, C₄- to C₂₀-cycloalkylalkyl, C₄- to C₂₀-alkylcycloalkyl, C₂- to C₂₀-alkoxyalkyl, C₂- to C₈-alkoxycarbonyl, C₁- to C₂₀-haloalkyl, aryl, C₇- to C₂₀-aralkyl or C₇- to C₂₀-alkylaryl, and
R^{2'} and R^{3'} , independently of one another, are hydrogen, C₁-to C₂₀-alkyl, which is unsubstituted or substituted by one to four heterocyclic radicals or by C₂- to C₈-carbalkoxy, carboxyl, C₁- to C₈-alkylamino and/or hydroxyl, or are C₂- to C₂₀-alkenyl, C₃- to C₂₀-cycloalkyl, C₄- to C₂₀-cycloalkylalkyl, C₄- to C₂₀-alkylcycloalkyl, C₂-to C₂₀-alkoxyalkyl, C₂- to C₈-alkoxycarbonyl, C₁-to C₂₀-haloalkyl, aryl, C₇- to C₂₀-aralkyl or C₇-to C₂₀-alkylaryl, or together are a C₄- to C₇-alkylene chain which is unsubstituted or substituted by one to five C₁-to C₄-alkyl groups,
with the proviso that R^{1'} is not methyl if R^{2'} and R^{3'} are simultaneously hydrogen.

3. A pentane 1,5-diamine of the formula I' as claimed in claim 2, in which R^{1'} is C₁- to C₄-alkyl, and R^{2'} and R^{3'}, independently of one another, are hydrogen or C₁- to C₈-alkyl, with the proviso that R^{2'} and R^{3'} are not simultaneously hydrogen if R^{1'} is methyl.

4. A pentane-1,5-diamine of the formula I' as claimed in claim 2, in which R^{1'} is ethyl, propyl, isopropyl, n-butyl, sec-butyl, isobutyl or tert-butyl, and R^{2'} and R^{3'}, independently of one another, are hydrogen or C₁-to C₈-alkyl.

5. A pentane-1,5-diamine of the formula I' as claimed in claim 2, in which R^{1'} is methyl and R^{2'} and R^{3'}, independently of one another, are hydrogen or C₁- to C₈-alkyl, with the proviso that R^{2'} and R^{3'} are not simultaneously hydrogen.

6. The use of a pentane-1,5-diamine I as claimed in claim 1 as a curing agent for epoxy resins.

7. The use of a pentane-1,5-diamine I' as claimed in any of claims 2 to 4 as a curing agent for epoxy resins.

8. The use of a pentane-1,5-diamine I as claimed in claim 1 as a component for polyamides.

9. The use of a pentane-1,5-diamine I' as claimed in any of claims 2 to 4 as a component for polyamides.

## Revendications

1. Procédé de préparation de pentane-1,5-diamines de formule générale I 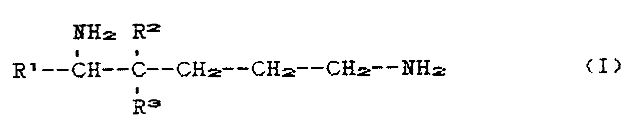 dans laquelle
R¹ représente un groupement alkyle en C₁-C₂₀ qui est éventuellement substitué par un à quatre restes hétérocycliques, carbalcoxy en C₂-C₈, carboxy, alkylamino en C₁-C₈ et/ou hydroxy, ou un groupement alcényle en C₂-C₂₀, cycloalkyle en C₃-C₂₀, cycloalkylalkyle en C₄-C₂₀, alkylcycloalkyle en C₄-C₂₀, alcoxyalkyle en C₂-C₂₀, alcoxycarbonyle en C₂-C₈, halogénoalkyle en C₁-C₂₀, aryle, aralkyle en C₇-C₂₀ ou alkylaryle en C₇-C₂₀, et
R², R³ représentent chacun, indépendamment l'un de l'autre, un atome d'hydrogène ou un groupement alkyle en C₁-C₂₀ qui est éventuellement substitué par un à quatre restes hétérocycliques, carbalcoxy en C₂-C₈, carboxy, alkylamino en C₁-C₈ et/ou hydroxy, ou un groupement alcényle en C₂-C₂₀, cycloalkyle en C₃-C₂₀, cycloalkylalkyle en C₄-C₂₀, alkylcycloalkyle en C₄-C₂₀, alcoxyalkyle en C₂-C₂₀, alcoxycarbonyle en C₁-C₈, halogénoalkyle en C₁-C₂₀, aryle, aralkyle en C₇-C₂₀ ou alkylaryle en C₇-C₂₀, ou sont mis en commun pour une chaîne alkylène en C₄-C₇ éventuellement substituée par un à cinq groupements alkyle en C₁-C₄,
à partir de γ-cyanocétones de formule générale II 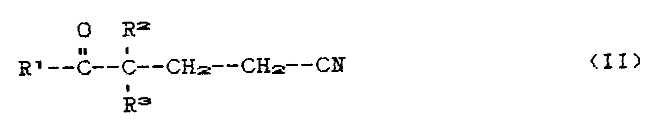 dans laquelle les substituants R¹, R² et R³ ont les mêmes significations que précédemment,
caractérisé en ce que, dans deux zones de réaction séparées l'une de l'autre dans l'espace,
a) on fait réagir les cyanocétones de formule II, dans une première zone de réaction, avec de l'ammoniac en excès en présence de catalyseurs hétérogènes acides, à des températures de 20 à 150°C et sous des pressions de 15 à 500 bar, et
b) on hydrogène les produits de réaction formés, dans une seconde zone de réaction, avec de l'hydrogène en présence d'ammoniac en excès et de catalyseurs contenant du cobalt, du nickel, du ruthénium, du palladium et/ou d'autres métaux précieux, éventuellement avec des composants basiques ou sur des supports basiques ou neutres, à des températures de 50 à 180°C et sous des pressions de 30 à 500 bar.

2. Pentane-1,5-diamines de formule générale I' 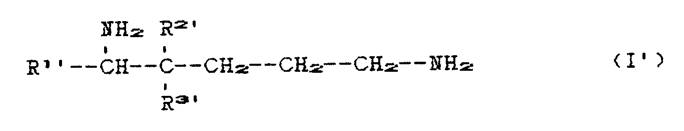 dans laquelle
R^{1'} représente un groupement alkyle en C₁-C₂₀ qui est éventuellement substitué par un à quatre restes hétérocycliques, carbalcoxy en C₂-C₈, carboxy, alkylamino en C₁-C₈ et/ou hydroxy, ou un groupement alcényle en C₂-C₂₀, cycloalkyle en C₃-C₂₀, cycloalkylalkyle en C₄-C₂₀, alkylcycloalkyle en C₄-C₂₀, alcoxyalkyle en C₂-C₂₀, alcoxycarbonyle en C₂-C₈, halogénoalkyle en C₁-C₂₀, aryle, aralkyle en C₇-C₂₀ ou alkylaryle en C₇-C₂₀, et
R^{2'}, R^{3'} représentent chacun, indépendamment l'un de l'autre, un atome d'hydrogène ou un groupement alkyle en C₁-C₂₀ qui est éventuellement substitué par un à quatre restes hétérocycliques, carbalcoxy en C₂-C₈, carboxy, alkylamino en C₁-C₈ et/ou hydroxy, ou un groupement alcényle en C₂-C₂₀, cycloalkyle en C₃-C₂₀, cycloalkylalkyle en C₄-C₂₀, alkylcycloalkyle en C₄-C₂₀, alcoxyalkyle en C₂-C₂₀, alcoxycarbonyle en C₂-C₈, halogénoalkyle en C₁-C₂₀, aryle, aralkyle en C₇-C₂₀ ou alkylaryle en C₇-C₂₀, ou sont mis en commun pour une chaîne alkylène en C₄-C₇ éventuellement substituée par un à cinq groupements alkyle en C₁-C₄,
étant spécifié que R^{1'} ne représente pas un groupement méthyle lorsque R^{2'} et R^{3'} représentent en même temps des atomes d'hydrogène.

3. Pentane-1,5-diamines de formule générale I' selon la revendication 2, dans lesquelles R^{1'} représente un groupement alkyle en C₁-C₄ et R^{2'}, R^{3'} représentent chacun, indépendamment l'un de l'autre, un atome d'hydrogène ou un groupement alkyle en C₁-C₈, étant spécifié que R^{2'} et R^{3'} ne sont pas simultanément des atomes d'hydrogène lorsque R^{1'} représente un groupement méthyle.

4. Pentane-1,5-diamines de formule générale I' selon la revendication 2, dans lesquelles R^{1'} représente un groupement éthyle, propyle, isopropyle, n-butyle, sec.-butyle, isobutyle ou tert.-butyle et R^{2'}, R^{3'} représentent chacun, indépendamment l'un de l'autre, un atome d'hydrogène ou un groupement alkyle en C₁-C₈.

5. Pentane-1,5-diamines de formule générale I' selon la revendication 2, dans lesquelles R^{1'} représente un groupement méthyle et R^{2'}, R^{3'} représentent chacun, indépendamment l'un de l'autre, un atome d'hydrogène ou un groupement alkyle en C₁-C₈, étant spécifié que R^{2'} et R^{3'} ne représentent pas simultanément des atomes d'hydrogène.

6. Utilisation des pentane-1,5-diamines I selon la revendication 1 comme durcisseurs pour des résines époxy.

7. Utilisation des pentane-1,5-diamines I' selon l'une quelconque des revendications 2 à 4 comme durcisseurs pour des résines époxy.

8. Utilisation des pentane-1,5-diamines I selon la revendication 1 comme composants pour des polyamides.

9. Utilisation des pentane-1,5-diamines I' selon l'une quelconque des revendications 2 à 4 comme composants pour des polyamides.
